# EUROPEAN PATENT SPECIFICATION

(11) **EP 2 381 929 B1**
(45) Date of publication and mention of the grant of the patent: **08.07.2015**
(21) Application number: 09836366.6
(22) Date of filing: 28.12.2009
(51) Int. Cl.: A61K 9/19, A61K 47/34

(54) **PHARMACEUTICAL COMPOSITION OF LYOPHILIZED FORMULATION AND PREPARATION METHOD OF THE SAME**
PHARMAZEUTISCHE ZUSAMMENSETZUNG EINER LYOPHILISIERTEN FORMULIERUNG UND HERSTELLUNGSVERFAHREN DAFÜR
COMPOSITION PHARMACEUTIQUE D'UNE FORMULE LYOPHILISÉE ET SA MÉTHODE D'ÉLABORATION

(30) Priority: 29.12.2008 KR 20080135414
(43) Date of publication of application: 02.11.2011
(73) Proprietor: Samyang Biopharmaceuticals Corporation, Seoul 110-470 (KR)
(72) Inventor: SEO, Min Hyo, Daejeon 302-782 (KR); LEE, Sang Jun, Dajeon 302-150 (KR)
(74) Representative: Ferreccio, Rinaldo
(86) International application number: PCT/KR2009/007844
(87) International publication number: WO 2010/077045

(56) References cited:
- WO-A1-2006/014067
- US-A1- 2003 187 148
- US-A1- 2005 129 731
- US-A1- 2008 299 205

## Description

### Technical Field

This disclosure relates to a pharmaceutical composition of a lyophilized formulation and a method for preparing the same.

### Background Art

Hydrogels have been used as drug delivery systems because of their biocompatibility. A drug is entrapped in the crosslinked hydrogel matrix and then released through the pore in the matrix by diffusion.

The early gel-based drug delivery system had thermoplastic or thermosetting properties. The thermoplastic system has drawn attentions because it quickly forms gel upon injection into the body as liquid due to the body temperature. However, the early gel-based drug delivery system is problematic in toxicity and irritation because an organic solvent has to be used.

Recently, a gel drug delivery system that can be prepared in aqueous solution was developed. Korean Patent Publication No. 2007-042159 discloses a multiblock poloxamer having the phase-transition property, which, upon administration into the body as dissolved in a solution of protein, peptide or analogs thereof, forms a solid gel phase due to the body temperature. The resultant multiblock poloxamer gel delays the release of peptide, which can be applied to prepare a sustained-release drug formulation.

With this method, microspheres may be prepared easily without having to use an organic solvent. As a result, the stability of peptide does not drop. Further, since the multiblock poloxamer is a biodegradable polymer, it is mostly degraded and excreted out of the body within one week after administration.

However, the multiblock poloxamer needs to be kept in a dry status to store it for a long period of time. The easiest applicable drying method is lyophilization. However, the known lyophilization technique for the multiblock poloxamer is restricted in commercialization because a very long time(maximum to 6 hours according to composition) is required for reconstitution following the lyophilization.

### Disclosure of Invention

### Technical Problem

Thus, this disclosure is directed to providing a pharmaceutical composition of a lyophilized formulation capable of remarkably reducing the reconstitution time.

It is also directed to providing a method for preparing the pharmaceutical composition of a lyophilized formulation.

### Solution to Problem

In one aspect, there is provided a method for preparing a pharmaceutical composition of a lyophilized formulation including a multiblock copolymer in which two or more ABA-type triblocks are covalently connected through biodegradable dicarboxylic linkage, wherein A represents a polyethylene oxide block and B represents a polypropylene oxide block, a polybutylene oxide block or a combination thereof, the multiblock copolymer being included in an amount of 1 to 7 wt% based on the total weight of solution prior to the lyophilized formulation.

In another aspect, there is provided a method for preparing a reconstituted solution of the pharmaceutical composition of a lyophilized formulation.

### Advantageous Effects of Invention

The disclosed pharmaceutical composition of a lyophilized formulation is advantageous in that reconstitution time may be remarkably reduced to such as 5 minutes to 1hour.

### Brief Description of Drawings

The above and other aspects, features and advantages of the disclosed exemplary embodiments will be more apparent from the following detailed description taken in conjunction with the accompanying drawings in which:

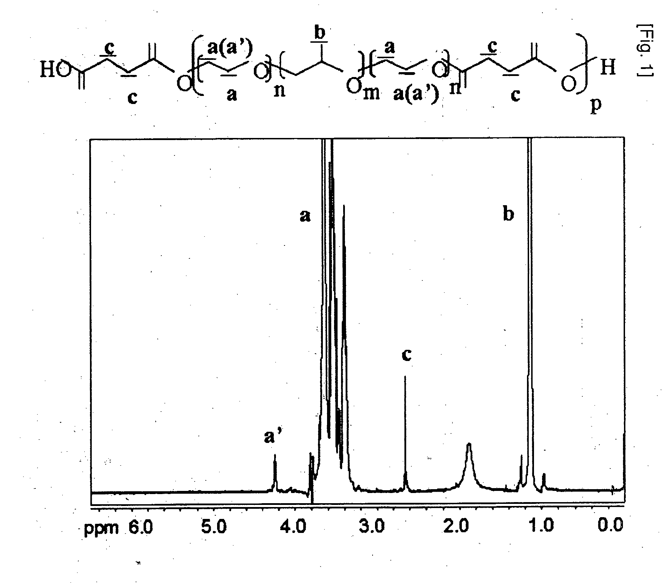

Fig. 1 shows ¹H-NMR spectrum of the multiblock copolymer prepared in Preparation Example 1.

### Best Mode for Carrying out the Invention

Exemplary embodiments now will be described more fully hereinafter with reference to the accompanying drawings, in which exemplary embodiments are shown. This disclosure may, however, be embodied in many different forms and should not be construed as limited to the exemplary embodiments set forth therein. Rather, these exemplary embodiments are provided so that this disclosure will be thorough and complete, and will fully convey the scope of this disclosure to those skilled in the art. In the description, details of well-known features and techniques may be omitted to avoid unnecessarily obscuring the presented embodiments.

The terminology used herein is for the purpose of describing particular embodiments only and is not intended to be limiting of this disclosure. As used herein, the singular forms "a","an" and "the" are intended to include the plural forms as well, unless the context clearly indicates otherwise. Furthermore, the use of the terms a, an, etc. does not denote a limitation of quantity, but rather denotes the presence of at least one of the referenced item. The use of the terms "first", "second", and the like does not imply any particular order, but they are included to identify individual elements. Moreover, the use of the terms first, second, etc. does not denote any order or importance, but rather the terms first, second, etc. are used to distinguish one element from another. It will be further understood that the terms "comprises" and/or "comprising" or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

Unless otherwise defined, all terms (including technical and scientific terms) used herein have the same meaning as commonly understood by one of ordinary skill in the art. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

According to an embodiment, a pharmaceutical composition of a lyophilized formulation comprises a multiblock copolymer in which two or more ABA-type triblocks are covalently connected through biodegradable dicarboxylic linkage, wherein A represents a polyethylene oxide (PEO) block and B represents a polypropylene oxide (PPO) block, a polybutylene oxide (PBO) block, or a block of copolymer of propylene oxide and butylene oxide(PPOBO), the multiblock copolymer being comprised in an amount of 1 to 7 wt% based on the total weight of solution prior to the lyophilized formulation.

Since the multiblock copolymer is biodegradable, long-term storage in aqueous solution state is difficult because it is hydrolyzed in aqueous solution. To solve this problem, the multiblock copolymer may be lyophilized to extend the storage period. Later, the lyophilized multiblock copolymer is reconstituted for use with the addition of aqueous solution. For conventient of use, a short reconstitution time is required.

When the multiblock copolymer is lyophilized, the reconstitution time of the lyophilized copolymer varies depending on the particular lyophilization method. The inventors have found that the reconstitution time increases abruptly as the concentration of the copolymer in the solution before the lyophilization increases and that the reconstitution time may be reduced by varying the lyophilization method. Thus, this disclosure is directed to reducing reconstitution time of a lyophilized formulation using a polymer undergoing phase transition depending on temperature. The multiblock copolymer may be comprised in an amount of 1 to 7 wt%, specifically 2 to 5 wt%, on the total weight of solution prior to the lyophilized formulation. Accordingly, the lyophilized formulation comprising the multiblock copolymer may be reconstituted to an aqueous solution at the concentration of 4 to 25 wt% in a very shot time such as 5 minutes to 1 hour, specifically 5 to 30minutes, more specifically 5 to 15minutes.

The terms used herein are defined as follows.

The term "multiblock" copolymer refers to a copolymer wherein a PEO block is linked to a PPO block, PBO block, or a block of copolymer of propylene oxide and butylene oxide(PPOBO), which is, in turn, linked to a PEO block and the resulting PEO-PPO (or PBO or PPOBO)-PEO blocks are connected through biodegradable dicarboxylic linkages. The copolymer of propylene oxide and butylene oxide(PPOBO) could a block or random copolymer.

The term "dicarboxylic linkage" refers to an ester linkage formed by the reaction with a dicarboxylic acid having two carboxylic groups in one molecule such as oxalic acid, malonic acid, succinic acid, adipic acid, and so on with a terminal OH group of a PEO-PPO or PBO, or PPOBO-PEO block. The dicarboxylic linkage may be provided by an alkyl dicarboxylic acid selected from a group consisting of oxalic acid, malonic acid, malic acid, succinic acid, glutaric acid, adipic acid, pimelic acid, suberic acid, azelaic acid, sebacic acid and dodecanoic acid. The dicarboxylic linkage may also be provided by an unsaturated dicarboxylic acid such as fumaric acid or maleic acid, or by an aryl dicarboxylic acid such as phthalic acid or terephthalic acid.

The term "biodegradable" refers to a property of a material that is degraded by hydrolysis or enzymatic action after being administered into the body, thereby being absorbed by the body or safely excreted out of the body.

The term "poloxamer" refers to a triblock PEO-PPO-PEO copolymer compound composed of a central hydrophobic block of PPO linked with two hydrophilic blocks of PEO by ether linkages, the copolymer having a weight average molecular weight ranging from 1,000 to 20,000 daltons, and both terminal groups being hydroxyl. Particularly, it includes the commercially available compounds poloxamer 188 (Pluronic® F68) and poloxamer 407 (Pluronic® F127).

The term "multiblock poloxamer" refers to a polymer formed as two or more "poloxamers" are connected through "dicarboxylic linkages" and having a weight average molecular weight of 40,000 daltons or above.

The term "sol-gel phase transition" to the phenomenon in which a material is present in a sol state below a transition temperature and is changed into a gel above the transition temperature, and if a temperature is lowered below the transition temperature, it is reversibly changed into the sol state. The critical temperature depends on the type of the polymer, the concentration of the polymer aqueous solution, the absence or presence of a salt, the proton concentration, and so on. The transition temperature may range from 5 to 37 °C, specifically from 25 to 35 °C.

And, the term "lyophilization" refers to a drying process by freezing a material dissolved in water below 0 °C and then reducing the surrounding pressure using a vacuum pump to allow the frozen water in the material to sublimate.

According to an embodiment, the multiblock copolymer may be represented by Chemical Formula 1:

Chemical Formula 1 M-X-O-[PEO-Y-PEO-C(=O)-R-C(=O)-O]ₙ-PEO-Y-PEO-O-X-M

wherein
PEO represents a polyethylene oxide block,
Y is PPO or PBO, or PPOBO, wherein PPO represents a polypropylene oxide block and PBO represents a polybutylene oxide block, PPOBO represents a block of copolymer of propylene oxide and butylene oxide,
X represents H or an anion group,
n represents an integer from 1 to 100,
R represents -(CH₂)ₘ- or aryl having Cₘ,
m represents an integer from 0 to 20,
m' represents an integer from 6 to 12, and
M represents H or a cation group, with the proviso that M and X are not H at the same time, and M is nonexistent when X is H.
X may be an anion group selected from a group consisting of -SO3-, -PO3²⁻ and-C(=O)-R-C(=O)-O-, and M may be a cation group selected from a group consisting of Li, Na, K, Ag, Au, Ca, Mg, Zn, Fe, Cu, Co and Ni.

According to another embodiment, the multiblock copolymer may have a weight average molecular weight ranging from 40,000 to 1,000,000 daltons, specifically from 40,000 to 500,000 daltons, and more specifically from 80,000 to 130,000 daltons.

According to an embodiment, the multiblock copolymer is synthesized as follows. First, 0.5 to 1.0 molar equivalent of dicarboxylic acid dichloride is slowly added to PEO-PPO (or PBO or PPOBO)-PEO, based on the terminal hydroxyl groups of PEO-PPO (or PBO or PPOBO)-PEO, over 6 hours. After reaction over 12 hours, 0.1 molar equivalent of PEO-PPO (or PBO or PPOBO)-PEO is added to the reaction solution and is reacted over 2 hours. Thus synthesized multiblock copolymer is precipitated in ether solvent and dissolved in methanol to separate the polymer. To thus the separated polymer, ether is slowly added to obtain a mixture of methanol/ether with a volume ratio of 1/1 to 1/20. Then, a multiblock copolymer with hydroxyl groups at both termini is precipitated and obtained. A more detailed synthesis method is disclosed in Korean Patent Publication No. 2007-042159.

The multiblock copolymer aqueous solution is present as a flowable liquid at low temperature (10 °C or below) and is changed into a gel above a critical temperature. The multiblock copolymer aqueous solution in liquid state could be administered to the human body using a normal syringe. When the polymer is administered to the human body, it is changed into a solid phase due to the body temperature. Therefore, by incluidng an active ingredient such as protein in the multiblock copolymer aqueous solution, a sustained-release effect of the ingredient may be attained because the ingredient is confined in the solid multiblock copolymer.

Accordingly, the disclosed lyophilized formulation may further comprise an active ingredient that may be delivered by the multiblock copolymer. The active ingredient may be but not limited to a bioactive agent having a pharmacological effect. In an embodiment, the active ingredient may be a protein or a peptide. For example, it may be one or more selected from a group consisting of growth hormone (GH), interferon (IFN), granulocyte colony-stimulating factor (G-CSF), granulocyte macrophage colony-stimulating factor (GM-CSF), erythropoietin (EPO), interleukin (IL), fibroblast growth factor, follicle-stimulating hormone (FSH), nerve growth factor (NGF), octreotide, insulin, insulin-like growth factor (IGF), calcitonin, tumor necrosis factor (TNF), vascular endothelial growth factor (VEGF), epidermal growth factor (EGF), platelet-derived growth factor (PDGF), bone morphogenetic protein (BMP), tissue plasminogen activator (TPA), thrombopoietin (TPO), tissue growth factor (TGF) and exenatide. The peptide or protein may be natural, synthetic, native, glycosylated, modified with a polymer such as PEG or biologically active fragments and analogs thereof. In an embodiment, the active ingredient is comprised in an amount of 1 to 50 wt% based on the total weight of the lypophilized formulation.

The composition of the lyophilized formulation may further comprise a pharmaceutical adjuvant such as preservative, stabilizer, wetting agent, salt to change the osmotic pressure, and/or buffer. It may also comprise other therapetuically active material.

In an embodiment, the composition may further comprise one or more buffer(s) selected from a group consisting of acetate, citrate, glycinate, phosphate and carbonate. A protein or peptide drug is sensitive to the composition of the formulation, particularly to pH, and may experience change in structure or decrease in activity. Accordingly, if a buffer is used instead of pure water when preparing the multiblock copolymer solution, the stability of the formulation may be improved.

In another embodiment, the composition may further comprise one or more biodegradable material(s) selected from a group consisting of collagen, gelatin, dextran, dextrin, hyaluronic acid and chitosan. The addition of the biodegradable material to the multiblock copolymer aqueous solution enables sustained release of the protein or peptide after the lyophilized formulation is administered into the body.

In an embodiment, the composition of the lyophilized formulation may further comprise a lyophilization aid. The lyophilization aid may be a sugar, a sugar alcohol, and mixtures thereof. The sugar may be at least one selected from lactose, maltose, sucrose, trehalose and a combination thereof. The sugar alcohol may be at least one selected from the mannitol, sorbitol, maltitol, xylitol, lactitol and a combination thereof. In another embodiment, the lyophilization aid is comprised in an amount of 1 to 50 wt%, specifically 1 to 30 wt%, based on the total weight of the lyophilized formulation.

The disclosure provides a method for preparing the pharmaceutical composition of the lyophilized formulation comprises the multiblock copolymer. In an embodiment, the method for preparing the composition comprises:
(a) dissolving a multiblock copolymer in a solvent at the concentration of 1 to 7 wt%, specifically 2 to 5 wt% based on the total weight of the resulting solution to obtain a polymer solution ; and
(b) lyophilizing the solution prepared in (a).

In an embodiment, the method for preparing the pharmaceutical composition may comprises further adding a protein or a peptide when dissolving the polymer in step (a).

In an embodiment, the concentration of the aqueous solution of the multiblock copolymer enabling sustained release of the active ingredient and allowing easier administration using a syringe is about 4 to 25 wt%. However; if an aqueous solution with such a concentration is lyophilized, a very long time will be necessary for reconstitution.

That is to say, the reconstitution time of the lyophilized formulation varies greatly depending on the concentration of the multiblock copolymer in the aqueous solution prior to lyophilization. To reduce the reconstitution time, a sufficient space should be ensured to allow infiltration of water into the lyophilized formulation. In addition, it is necessary to decrease the viscosity of the dissolved multiblock copolymer at the surface at which it contacts with water. In this disclosure, the concentration of the multiblock copolymer in the aqueous solution before lyophilization is controlled in order to decrease the viscosity during the reconstitution. The concentration control also allows a larger lyophilized cake pore.

According to this disclosure, the concentration of the multiblock copolymer in the aqueous solution before lyophilization is 1 to 7 wt%, specifically 2 to 5 wt%. If the concentration of the multiblock copolymer exceeds 7 wt%, when the lyophilized multiblock copolymer is reconstituted by adding water, the multiblock copolymer which beings to be dissolved at the contact surface with water becomes a viscous liquid. This viscous liquid inhibits the penetration of water into the cake below it. As a result, the reconstitution time of the lyophilized formulation may increase. On the other hand, the reconstitution time may also increase if the concentration of the multiblock copolymer is below 1 wt%. If the concentration of the multiblock copolymer is below 1 wt%, the lyophilized multiblock copolymer turns into a porous cake. Due to the pores formed during the lyophilization, only part of the polymer contacts with water, and the remaining may not contact with water. As a result, the multiblock copolymer is not dissolved, or the reconstitution time may increase remarkably.

In an embodiment, the lyophilized formulation may be reconstituted in distilled water for injection, 5% glucose solution, physiological saline, or the like at a concentration of 4 to 25 wt%, specifically 7 to 13 wt% for administration into the human body. If the reconstituted concentration of the multiblock copolymer exceeds 25 wt%, it is hard to reconstitute the polymer and inject the polymer solution by a syringe. On the other hand, if the reconstituted concentration is below 4 wt%, gel does not form formation with the polymer.

### Mode for the Invention

The examples and experiments will now be described. The following examples and experiments are for illustrative purposes only and not intended to limit the scope of this disclosure.

### Preparation Example 1: Synthesis of multiblock copolymer comprising poloxamer 407 using succinyl dichloride

10 g of poloxamer 407 (Pluronic® F127, BASF, molecular weight: 12,500 daltons, PEO:PPO = 101:56) is added to a 100 mL 1-neck round bottom flask. After heating in an oil bath heated at 120 °C, water included in the polymer is removed for 2 hours under reduced pressure. After restoring pressure, 100 mL of acetonitrile is added to the flask while flowing in nitrogen, with the reaction temperature maintained at 100 °C. A Dean-Stark apparatus and a condenser are installed at the reaction flask. 20 mL of acetonitrile distilled out is removed using the Dean-Stark apparatus. After the water is completely removed, 1 molar equivalent of succinyl dichloride is added to a reservoir of the Dean-Stark apparatus. After performing reaction for 24 hours, in order to substitute the terminal groups of the resultant poloxamer oligomer with carboxylic groups, 96 ul of succinyl chloride is added again to the reservoir of the Dean-Stark apparatus. Thus synthesized poloxamer oligomer is precipitated in 1 L of diethyl ether and filtered to obtain the product (8.2 g). The obtained product is dissolved again in 16 mL of methanol, precipitated in diethyl ether and purified by filtration. After repeating this procedure twice, the product is dried in vacuum. Poloxamer oligomer (5.7 g) with a narrow molecular weight distribution is obtained.

GPC reveals that the resultant multiblock copolymer has a weight average molecular weight of 90,700 daltons, and ¹H-NMR confirms its synthesis (Fig. 1).

### Comparative Example 1

The multiblock copolymer prepared in Preparation Example 1 is dissolved in distilled water at concentrations ranging from 7 to 15 wt% and lyophilized at -20 °C. To each of the lyophilized multiblock copolymer, distilled water is added such that the concentration of the multiblock copolymer is the same as before the lyophilization. Then, the time until complete dissolution is measured. The result is given in Table 1.

**[Table 1]**

| Concentration of polymer solution before lyophilization (wt%) | Concentration of polymer after reconstitution (wt%) | Dissolution time |
|---|---|---|
| 15 | 15 | 6 hr |
| 13 | 13 | 3 hr 45 min |
| 11 | 11 | 3 hr |
| 9 | 9 | 1 hr 40 min |
| 7 | 7 | 35 min |

As seen in Table 1, the dissolution time rapidly increases as the concentration of the multiblock copolymer in the aqueous solution before lyophilization increases. In case of the 15% multiblock copolymer aqueous solution, a very long period of time is required until the polymer is completely dissolved.

### Comparative Example 2

The multiblock copolymer prepared in Preparation Example 1 is dissolved in a pH 5.0, 0.1 M acetate buffer solution at a concentration of 7-12 wt% and lyophilized. To the lyophilized multiblock copolymer, different amount of distilled water is added. Then, the time until complete dissolution is measured. The result is given in Table 2.

**[Table 2]**

| Concentration of polymer solution before lyophilization (wt%) | Concentration of polymer after reconstitution (wt%) | Dissolution time |
|---|---|---|
| 12 | 12 | 5 hr 15 min |
| 11 | 11 | 4 hr |
| 9 | 9 | 2 hr |
| 7 | 7 | 45 min |

As seen in Table 1 and 2, when a lyophilization is proceed using acetate buffer, the dissolution time is relatively increased.

### Comparative Example 3

Hyalurnoic acid is added to 7∼12 wt% multiblock copolymer aqueous solution in an amount of 1 wt% based on the polymer prepared in Preparation Example 1, and the resulting solution is lyophilized. To the lyophilized multiblock copolymer, different amount of distilled water is added. Then, the time until complete dissolution is measured. The result is given in Table 3.

**[Table 3]**

| Concentration of polymer solution before lyophilization (wt%) | Concentration of polymer after reconstitution (wt%) | Dissolution time |
|---|---|---|
| 12 | 12 | 5 hr 30 min |
| 11 | 11 | 4 hr 35 min |
| 9 | 9 | 2 hr 40 min |
| 7 | 7 | 55 min |

As seen in Table 1 and 3, when hyalurnoic acid is added to the multiblock poloxamer, the dissolution time is relatively increased.

### Example 1

The multiblock copolymer prepared in Preparation Example 1 is prepared into 2 wt% aqueous solution and lyophilized. To the lyophilized multiblock copolymer, different amount of distilled water is added. Then, the time until complete dissolution is measured. The result is given in Table 4.

**[Table 4]**

| Concentration of polymer solution before lyophilization (wt%) | Concentration of polymer after reconstitution (wt%) | Dissolution time (min) |
|---|---|---|
| 2 | 12 | 9 |
| 2 | 11 | 7 |
| 2 | 10 | 6 |
| 2 | 9 | 5 |
| 2 | 8 | 5 |
| 2 | 7 | 5 |

As seen in Table 4, if the concentration of the polymer before lyophilization is adjusted to 2 wt%, there is no significant increase in the dissolution time when the concentration of polymer after reconstitution is increased. Even though lyophilized polymer solution is dissolved to reach the higher the polymer concentration of 12 wt%, there is no significant change in the dissolution time. The dissolution time to prepare the concentration of 7 to 12 wt% of the multiblock copolymer solution is significantly decreased as compared to Comparative Example 1.

### Example 2

The multiblock copolymer prepared in Preparation Example 1 is prepared into 5 wt% aqueous solution and lyophilized. To the lyophilized multiblock copolymer, different amount of distilled water is added. Then, the time until complete dissolution is measured. The result is given in Table 5.

**[Table 5]**

| Concentration of polymer solution before lyophilization (wt%) | Concentration of polymer after reconstitution (wt%) | Dissolution time (min) |
|---|---|---|
| 5 | 12 | 9 |
| 5 | 11 | 9 |
| 5 | 10 | 8 |
| 5 | 9 | 8 |
| 5 | 8 | 6 |
| 5 | 7 | 6 |

As seen in Table 5, if the concentration of the polymer before lyophilization is adjusted to 5 wt%, there is no significant increase in the dissolution time when the concentration of polymer after reconstitution is increased. Even though lyophilized polymer solution is dissolved to reach the higher the polymer concentration of 12wt%, there is no significant change in the dissolution time. The dissolution time to prepare the concentration of 7 to 12wt% of the multiblock copolymer solution is significantly decreased as compared to Comparative Example 1.

### Example 3

The multiblock copolymer prepared in Preparation Example 1 is dissolved in a pH 5.0, 0.1 M acetate buffer solution at a concentration of 5 wt% and lyophilized. To the lyophilized multiblock copolymer, different amount of distilled water is added. Then, the time until complete dissolution is measured. The result is given in Table 6.

**[Table 6]**

| Concentration of polymer before lyophilization (wt%) | Concentration of polymer after reconstitution (wt%) | Dissolution time (min) |
|---|---|---|
| 5 | 12 | 13 |
| 5 | 11 | 12 |
| 5 | 10 | 10 |
| 5 | 9 | 10 |
| 5 | 8 | 7 |
| 5 | 7 | 7 |

If a salt is added to the multiblock copolymer aqueous solution, the dissolution time increases as seen in Comparative Example 2. However, in spite of the addition of salt, the dissolution time does not change rapidly in this Example.

### Example 4

Hyalurnoic acid is added to 5 wt% multiblock copolymer aqueous solution in an amount of 1 wt% based on the polymer, and the resulting solution is lyophilized. To the lyophilized multiblock copolymer, different amount of distilled water is added. Then, the time until complete dissolution is measured. The result is given in Table 7.

**[Table 7]**

| Concentration of polymer before lyophilization (wt%) | Concentration of polymer after reconstitution (wt%) | Dissolution time (min) |
|---|---|---|
| 5 | 12 | 15 |
| 5 | 11 | 15 |
| 5 | 10 | 13 |
| 5 | 9 | 11 |
| 5 | 8 | 9 |
| 5 | 7 | 8 |

Addition of hyalurnoic acid results in increased the dissolution time of the polymer aqueous solution as seen in Comparative Example 3. However, in spite of the addition of hyalurnoic acid, the dissolution time does not change rapidly in this Example.

As demonstrated through the foregoing examples, if the concentration of the polymer in the aqueous soluiton is adjusted to 1 to 7 wt% before lyophilization, the lyophilized cake has larger and more pores. As a result, the distilled water added to dissolve the lyophilized polymer is absorbed well and the viscosity of the polymer aqueous solution is decreased. Consequently, water penetrates well into the pores, and the dissolution time is decreased.

### Industrial Applicability

The disclosed composition is capable of reducing reconstitution time of lyophilized sustained-release formulations, and may be utilized in pharmaceutical and other fields.

## Claims

1. A method for preparing a pharmaceutical composition of a lyophilized formulation comprising a multiblock copolymer in which two or more ABA-type triblocks are covalently connected through biodegradable dicarboxylic linkage, wherein A represents a polyethylene oxide block and B represents a polypropylene oxide block, a polybutylene oxide block, or a block of copolymer of propylene oxide and butylene oxide, comprising:
- dissolving the multiblock copolymer in a solvent at the concentration of 1 to 7 wt% based on the total weight of the resulting solution to obtain a polymer solution; and
- lyophilizing the solution.

2. The method for preparing the pharmaceutical composition of a lyophilized formulation according to claim 1, wherein, in said dissolving the multiblock copolymer, 2 to 5 wt% of the multiblock copolymer is dissolved in a solvent, based on the total weight of the resulting solution.

3. The method for preparing the pharmaceutical composition of a lyophilized formulation according to claim 1, wherein a protein or a peptide is further dissolved with dissolving the multiblock copolymer.

4. The method for preparing the pharmaceutical composition of a lyophilized formulation according to claim 1, wherein the multiblock copolymer is represented by Chemical Formula 1:
Chemical Formula 1 M-X-O-[PEO-Y-PEO-C(=O)-R-C(=O)-O]ₙ-PEO-Y-PEO-O-X-M
wherein
PEO represents a polyethylene oxide block,
Y is PPO or PBO, or PPOBO, wherein PPO represents a polypropylene oxide block and PBO represents a polybutylene oxide block, PPOBO represents a block of copolymer of propylene oxide and butylene oxide,
X represents H or an anion group,
n-represents an integer from 1 to 100,
R represents -(CH₂)ₘ- or aryl having C_{m'},
m represents an integer from 0 to 20,
m' represents an integer from 6 to 12, and
M represents H or a cation group, with the proviso that M and X are not H at the same time, and M is nonexistent when X is H.

5. The method for preparing the pharmaceutical composition of a lyophilized formulation according to claim 1, wherein the multiblock copolymer has a weight average molecular weight ranging from 40,000 to 1,000,000 daltons.

6. The method for preparing the pharmaceutical composition of a lyophilized formulation according to claim 1, wherein said composition further comprises one or more buffer(s) selected from a group consisting of acetate, citrate, glycinate, phosphate and carbonate.

7. The method for preparing the pharmaceutical composition of a lyophilized formulation according to claim 1, wherein said composition further comprises one or more biodegradable material(s) selected from a group consisting of collagen, gelatin, dextrari, dextrin, hyaluronic acid and chitosan.

8. A method for preparing a reconstituted solution of a pharmaceutical composition of a lyophilized formulation comprising a multiblock copolymer in which two or more ABA-type triblocks are covalently connected through biodegradable dicarboxylic linkage, wherein A represents a polyethylene oxide block and B represents a polypropylene oxide block, a polybutylene oxide block, or a block of copolymer of propylene oxide and butylene oxide, said method comprising:
- dissolving the multiblock copolymer in a solvent at the concentration of 1 to 7 wt% based on the total weight of the resulting solution to obtain a polymer solution; and
- lyophilizing the solution;
wherein the lyophilized multiblock copolymer is reconstituted to an aqueous solution at the concentration ranging from 4 to 25 wt% based on the total aqueous solution.

## Patentansprüche

1. Verfahren zur Herstellung einer pharmazeutischen Zusammensetzung einer lyophilisierten Formulierung umfassend ein Multiblockcopolymer, in dem zwei oder mehrere ABA-Typ-Triblöcke über eine biologisch abbaubare Dicarbonylverbindung kovalent miteinander verknüpft sind, wobei A einen Polyethylenoxidblock darstellt und B einen Polypropylenoxidblock, einen Polybutylenoxidblock oder einen Block eines Copolymers von Propylenoxid und Butylenoxid darstellt, umfassend:
- Auflösen des Multiblockcopolymers in einem Lösungsmittel bei einer Konzentration von 1 bis 7 Gew.-%, basierend auf dem Gesamtgewicht der sich ergebenden Lösung, um eine Polymerlösung zu erhalten; und
- Lyophilisieren der Lösung.

2. Verfahren zur Herstellung der pharmazeutischen Zusammensetzung einer lyophilisierten Formulierung gemäß Anspruch 1, wobei beim Auflösen des Multiblockcopolymers 2 bis 5 Gew.-% des Multiblockcopolymers, basierend auf dem Gesamtgewicht der sich ergebenden Lösung, in einem Lösungsmittel aufgelöst sind.

3. Verfahren zur Herstellung der pharmazeutischen Zusammensetzung einer lyophilisierten Formulierung gemäß Anspruch 1, wobei ein Protein oder ein Peptid beim Auflösen des Multiblockcopolymers zusätzlich aufgelöst wird.

4. Verfahren zur Herstellung der pharmazeutischen Zusammensetzung einer lyophilisierten Formulierung gemäß Anspruch 1, wobei das Multiblockcopolymer durch die chemische Formel 1 dargestellt wird:
Chemische Formel 1 M-X-O-[PEO-Y-PEO-C(=O)-R-C(=O)-O]ₙ-PEO-Y-PEO-O-X-M
wobei
PEO einen Polyethylenoxidblock darstellt,
Y PPO oder PBO oder PPOBO ist, wobei PPO einen Polypropylenoxidblock darstellt und PBO einen Polybutylenoxidblock
darstellt, PPOBO einen Block eines Copolymers von Propylenoxid und
Butylenoxid darstellt,
X H oder eine Anionengruppe darstellt,
n eine ganze Zahl zwischen 1 bis 100 darstellt,
R -(CH₂)ₘ- oder ein Aryl mit C_{m'} darstellt,
m eine ganze Zahl zwischen 0 bis 20 darstellt,
m' einen ganze Zahl zwischen 6 bis 12 darstellt und
M H oder eine Kationengruppe darstellt, mit der Maßgabe, dass M und
X nicht gleichzeitig H sind und M nicht vorhanden ist, wenn X H ist.

5. Verfahren zur Herstellung der pharmazeutischen Zusammensetzung einer lyophilisierten Formulierung gemäß Anspruch 1, wobei das Multiblockcopolymer ein mittleres Molekulargewicht hat, das von 40.000 bis 1.000.000 Dalton reicht.

6. Verfahren zur Herstellung der pharmazeutischen Zusammensetzung einer lyophilisierten Formulierung gemäß Anspruch 1, wobei die Zusammensetzung zusätzlich ein oder mehrere Puffer, ausgewählt aus der Gruppe bestehend aus Acetat, Citrat, Glycinat, Phosphat und Carbonat, umfasst.

7. Verfahren zur Herstellung der pharmazeutischen Zusammensetzung einer lyophilisierten Formulierung gemäß Anspruch 1, wobei die Zusammensetzung zusätzlich ein oder mehrere biologisch abbaubare(s) Material(ien), ausgewählt aus der Gruppe bestehend aus Kollagen, Gelatine, Dextran, Dextrin, Hyaluronsäure und Chitosan, umfasst.

8. Verfahren zur Herstellung einer wiederhergestellten Lösung einer pharmazeutischen Zusammensetzung einer lyophilisierten Formulierung umfassend ein Multiblockcopolymer, in dem zwei oder mehrere ABA-Typ-Triblöcke über eine biologisch abbaubare Dicarbonylverbindung kovalent miteinander verknüpft sind, wobei A einen Polyethylenoxidblock darstellt und B einen Polypropylenoxidblock, einen Polybutylenoxidblock oder einen Block eines Copolymers von Propylenoxid und Butylenoxid darstellt, das Verfahren umfassend:
- Auflösen des Multiblockcopolymers in einem Lösungsmittel bei einer Konzentration von 1 bis 7 Gew.-%, basierend auf dem Gesamtgewicht der sich ergebenden Lösung, um eine Polymerlösung zu erhalten; und
- Lyophilisieren der Lösung;
wobei das lyophilisierte Multiblockcopolymer wieder zu einer wässrigen Lösung mit der Konzentration, die von 4 bis 25 Gew.-% reicht, basierend auf der gesamten wässrigen Lösung, hergestellt wird.

## Revendications

1. Procédé de préparation d'une composition pharmaceutique d'une formulation lyophilisée comprenant un copolymère multiséquencé dans lequel deux triséquences de type ABA ou plus sont reliées de manière covalente par une liaison dicarboxylique biodégradable, où A représente une séquence d'oxyde de polyéthylène et B représente une séquence d'oxyde de polypropylène, une séquence d'oxyde de polybutylène ou une séquence d'un copolymère d'oxyde de propylène et d'oxyde de butylène, consistant à :
- dissoudre le copolymère multiséquencé dans un solvant à la concentration de 1 à 7 % en poids par rapport au poids total de la solution résultante afin d'obtenir une solution de polymère ; et
- lyophiliser la solution.

2. Procédé de préparation de la composition pharmaceutique d'une formulation lyophilisée selon la revendication 1, dans lequel, pendant ladite dissolution du copolymère multiséquencé, de 2 à 5 % en poids du copolymère multiséquencé sont dissous dans un solvant, par rapport au poids total de la solution résultante.

3. Procédé de préparation de la composition pharmaceutique d'une formulation lyophilisée selon la revendication 1, dans lequel une protéine ou un peptide est en outre dissous lors de la dissolution du copolymère multiséquencé.

4. Procédé de préparation de la composition pharmaceutique d'une formulation lyophilisée selon la revendication 1, dans lequel le copolymère multiséquencé est représenté par la formule chimique 1 :
Formule chimique 1 M-X-O-[PEO-Y-PEO-C(=O)-R-C(=O)-O]ₙ-PEO-Y-PEO-O-X-M
où
PEO représente une séquence d'oxyde de polyéthylène,
Y est PPO ou PBO, ou PPOBO, où PPO représente une séquence d'oxyde de polypropylène et PBO représente une séquence d'oxyde de polybutylène, PPOBO représente une séquence d'un copolymère d'oxyde de propylène et d'oxyde de butylène,
X représente H ou un groupe anionique,
n représente un nombre entier de 1 à 100,
R représente -(CH₂)ₘ- ou un groupe aryle contenant C_{m'},
m représente un nombre entier de 0 à 20 ;
m' représente un nombre entier de 6 à 12, et
M représente H ou un groupe cationique, à condition que M et X ne soient pas H en même temps et que M ne soit pas non existant lorsque X est H.

5. Procédé de préparation de la composition pharmaceutique d'une formulation lyophilisée selon la revendication 1, dans lequel le copolymère multiséquencé a un poids moléculaire moyen en poids compris dans la plage allant de 40 000 à 1 000 000 daltons.

6. Procédé de préparation de la composition pharmaceutique d'une formulation lyophilisée selon la revendication 1, dans lequel ladite composition comprend en outre un ou plusieurs tampons choisis dans le groupe constitué par un acétate, un citrate, un glycinate, un phosphate et un carbonate.

7. Procédé de préparation de la composition pharmaceutique d'une formulation lyophilisée selon la revendication 1, dans lequel ladite composition comprend en outre une ou plusieurs substances biodégradables choisies dans le groupe constitué par le collagène, la gélatine, le dextrane, la dextrine, l'acide hyaluronique et le chitosane.

8. Procédé de préparation d'une solution reconstituée d'une composition pharmaceutique d'une formulation lyophilisée comprenant un copolymère multiséquencé dans lequel deux triséquences de type ABA ou plus sont reliées de manière covalente par une liaison dicarboxylique biodégradable, où A représente une séquence d'oxyde de polyéthylène et B représente une séquence d'oxyde de polypropylène, une séquence d'oxyde de polybutylène ou une séquence d'un copolymère d'oxyde de propylène et d'oxyde de butylène, consistant à :
- dissoudre le copolymère multiséquencé dans un solvant à la concentration de 1 à 7 % en poids par rapport au poids total de la solution résultante pour obtenir une solution de polymère ; et
- lyophiliser la solution ;
dans lequel le copolymère multiséquencé lyophilisé est reconstitué en une solution aqueuse à la concentration comprise dans la plage allant de 4 à 25 % en poids par rapport à la solution aqueuse totale.
